# EUROPÄISCHE PATENTSCHRIFT

(11) **EP 3 179 978 B1**
(45) Veröffentlichungstag und Bekanntmachung des Hinweises auf die Patenterteilung: **13.06.2018**
(21) Anmeldenummer: 15745497.6
(22) Anmeldetag: 05.08.2015
(51) Int. Cl.: A61K 8/26, A61K 8/28, A61Q 15/00, A61K 8/02, A61K 8/06, A61K 8/891

(54) **SCHWEISSHEMMENDE EMULSIONEN MIT GERINGER ADHÄSIVITÄT**
ANTITRANSPIRANT EMULSION WITH LOW ADHESIVABILITY
EMULSION ANTITRANSPIRANTE AVEC UNE ADHÉSIVIBILITÉ BASSE

(30) Priorität: 13.08.2014 DE 102014216039
(43) Veröffentlichungstag der Anmeldung: 21.06.2017
(73) Patentinhaber: Henkel AG & Co. KGaA, 40589 Düsseldorf (DE)
(72) Erfinder: DÖRING, Thomas, 41540 Dormagen (DE); SCHEVARDO, Natascha, 40699 Erkrath (DE)
(86) Internationale Anmeldenummer: PCT/EP2015/068063
(87) Internationale Veröffentlichungsnummer: WO 2016/023801

(56) Entgegenhaltungen:
- WO-A1-2006/063726
- DE-A1-102005 024 595
- DE-A1-102012 223 197

## Beschreibung

Die vorliegende Anmeldung betrifft wasserhaltige schweißhemmende Zusammensetzungen, synonym auch als Antitranspirant-Zusammensetzungen bezeichnet, die zur Applikation auf die Haut mit einem Rollapplikator geeignet sind und eine verringerte Adhäsivität auf der Haut aufweisen.

Es gibt zahlreiche Möglichkeiten, schweißhemmende Zusammensetzungen auf die Haut aufzutragen. Formstabile Stiftmassen werden aus einem Stiftspender heraus über die Haut gestrichen, bis eine wirksame Menge aufgetragen ist. Auch Gele und Cremes können mit stiftähnlichen Dispensern, die mit einer Dispenseroberfläche über die Haut gestrichen werden, aufgetragen werden. Insbesondere für schweißhemmende und/oder deodorierende Zusammensetzungen für den Achselbereich wurden zahlreiche verschiedene Applikationsformen entwickelt, neben den bereits genannten vor allem die treibgashaltigen und treibgasfreien Sprays und die Roll-on-Zusammensetzungen. Bei letzteren wird eine leicht verdickte Flüssigkeit aus einem Vorratsbehälter über eine drehbar gelagerte Kugel durch Rollen über die Haut appliziert. Schweißhemmende Roll-on-Zusammensetzungen können wasserfrei und ölbasiert sein; beispielsweise ist der ölbasierte Sud üblicher Antitranspirant-Sprays auch zur Darreichung als Roll-on geeignet. Hierbei liegt der schweißhemmende Wirkstoff als suspendiertes Pulver in einem Öl vor, das zur Verhinderung des Absetzens der Pulverpartikel mit einem lipophilen Geliermittel verdickt ist. Derartige Roll-ons sind allerdings im Markt kaum vertreten. Übliche schweißhemmende Roll-on-Zusammensetzungen sind wasserbasiert, das heißt, sie enthalten ca. 50 Gew.-% und mehr ihres Gesamtgewichts an Wasser. Der Antitranspirant-Wirkstoff, üblicherweise eine schweißhemmende Aluminium- oder Aluminium-Zirconium-Verbindung, liegt in gelöster Form vor.

Antitranspirantien für Roll-on Applikationen sind häufig vom Emulsionstyp. Bei diesen Produkten klagen Verbraucher häufig über ein klebriges Hautgefühl und eine zu langsame Trocknungsgeschwindigkeit. Daher gerät das auf der Haut getragene Textil meist in Kontakt mit der noch feuchten Produktmasse. Materialien wie Baumwolle saugen bei diesem Kontakt den Produktrückstand auf, so dass weniger Wirkstoff auf der Haut zur Verfügung steht. Die Wirkung als Antitranspirant und Deodorant ist daher vermindert und es kommt darüber hinaus zur unerwünschten Fleckenbildung auf dem Textil. Üblicherweise enthalten Wasser-basierte Antitranspirantien für Roll-on Applikationen einen relativ hohen Anteil an Ethanol, um das Antrocknen auf der Haut zu beschleunigen. Da die Hygieneroutine vieler Verbraucher das regelmäßige Rasieren der Achselhöhlen vor der Applikation eines Antitranspirants oder Deodorants vorsieht, führt die Applikation eines Ethanol-haltigen Mittels auf frisch rasierte Haut zu einem starken Brennen. Bei regelmäßiger Anwendung trocknen solche Mittel die Haut aus und machen sie so sensibel und reizbar DE102012223197, DE102005024595, WO2006063726 offenbaren verschiedene Zusammensetzungen für Antitranspirantien. Eine Aufgabe der vorliegenden Anmeldung war es daher, wasserbasierte Antitranspirant-Roll-ons mit geringer Adhäsivität und schnellerer Antrocknung bereitzustellen, die möglichst keine Haut reizenden Inhaltsstoffe, wie Ethanol, enthalten.

Überraschend wurde gefunden, dass die vorstehende Aufgabe gelöst wird durch den Zusatz einer Ölphase mit spezifischen Inhaltsstoffen.

Gegenstand der vorliegenden Anmeldung ist daher eine Antitranspirant-Zusammensetzung zur Roll-on-Applikation, enthaltend:
a) Wasser,
   b) mindestens eine schweißhemmende Aluminium- oder Aluminium-Zirconium-Verbindung,
   c) eine Ölphase, enthaltend:
      i) mindestens ein Siliconelastomer,
      ii) mindestens ein unter Normalbedingungen flüssiges Siliconöl, ausgewählt aus Dimethicone und Cyclomethicone, sowie
      iii) mindestens ein nur aus C und H bestehendes Kohlenwasserstofföl,
   wobei die Zusammensetzung kein Copolymer, bestehend aus mindestens zwei Monomeren, ausgewählt aus der Gruppe, die gebildet wird aus Ethylen, Propylen, Buten und Styren, enthält. Weitere bevorzugte Ausführungsformen der erfindungsgemäßen Zusammensetzungen sind den Unteransprüchen zu entnehmen.

### Wassergehalt

Die erfindungsgemäßen Zusammensetzungen enthalten bevorzugt 40 - 90 Gew.-%, besonders bevorzugt 50 - 85 Gew.-%, außerordentlich bevorzugt 60 - 80 Gew.-%, weiter außerordentlich bevorzugt 65 - 75 Gew.-% Wasser, jeweils bezogen auf das Gesamtgewicht der Zusammensetzung. Mit "Wasser" ist im Sinne der vorliegenden Anmeldung "freies Wasser" gemeint, also Wasser, das nicht in Form von Kristallwasser, Hydratationswasser oder ähnlich molekular gebundenem Wasser in der Antitranspirant-Zusammensetzung enthalten ist. Der Gehalt an Kristallwasser, Hydratationswasser oder ähnlich molekular gebundenem Wasser, der in den eingesetzten Bestandteilen, insbesondere in den schweißhemmenden Wirkstoffen, enthalten ist, stellt im Sinne der vorliegenden Anmeldung kein freies Wasser dar. Freies Wasser ist solches Wasser, das beispielsweise als Lösemittel oder als Lösemittelbestandteil anderer Wirkstoffe in der erfindungsgemäßen Zusammensetzung enthalten ist.

"Normalbedingungen" sind im Sinne der vorliegenden Anmeldung eine Temperatur von 20 °C (Maßbezugstemperatur) und ein Druck von 1013 mbar. Schmelzpunktangaben beziehen sich ebenfalls auf einen Druck von 1013 mbar.

### Schweißhemmende Aluminium- oder Aluminium-Zirconium-Verbindung

Besonders bevorzugte Antitranspirant-Wirkstoffe sind ausgewählt aus Aluminiumchlorhydrat, insbesondere Aluminiumchlorhydrat mit der allgemeinen Formel [Al₂(OH)₅Cl · 1-6 H₂O]ₙ, bevorzugt [Al₂(OH)₅Cl · 2-3 H₂O]ₙ, das in nicht-aktivierter oder in aktivierter (depolymerisierter) Form vorliegen kann, sowie Aluminiumchlorhydrat mit der allgemeinen Formel [Al₂(OH)₄Cl₂ · 1-6 H₂O]ₙ, bevorzugt [Al₂(OH)₄Cl₂ · 2-3 H₂O]ₙ, das in nicht-aktivierter oder in aktivierter (depolymerisierter) Form vorliegen kann.

Die Herstellung bevorzugter Antitranspirant-Wirkstoffe ist beispielsweise in US 3887692, US 3904741, US 4359456, GB 2048229 und GB 1347950 offenbart.

Weiterhin bevorzugt sind Aluminiumsesquichlorhydrat, Aluminiumdichlorhydrat, Aluminiumchlorhydrex-Propylenglykol (PG) oder Aluminiumchlorhydrex-Polyethylenglykol (PEG), Aluminium- oder Aluminiumzirkonium-Glycol-Komplexe, z. B. Aluminium- oder Aluminiumzirkonium-Propylenglycol-Komplexe, Aluminiumsesquichlorhydrex-PG oder Aluminiumsesquichlorhydrex-PEG, Aluminium-PG-dichlorhydrex oder Aluminium-PEG-dichlorhydrex, Aluminiumhydroxid, weiterhin ausgewählt aus den Aluminiumzirconiumchlorhydraten, wie Aluminiumzirconiumtrichlorhydrat, Aluminiumzirconiumtetrachlorhydrat, Aluminiumzirconiumpentachlorhydrat, Aluminiumzirconiumoctachlorhydrat, den Aluminium-Zirkonium-Chlorohydrat-Glycin-Komplexen wie Aluminiumzirconiumtrichlorhydrexglycin, Aluminiumzirconiumtetrachlorhydrexglycin, Aluminiumzirconiumpentachlorhydrexglycin, Aluminiumzirconiumoctachlorhydrexglycin, Kaliumaluminiumsulfat (KAl(SO₄)₂ · 12 H₂O, Alaun), dehydratisiertem Alaun (KAl(SO₄)₂ mit null bis 11 Mol Kristallwasser), Aluminiumundecylenoylcollagenaminosäure, Natriumaluminiumlactat + Aluminiumsulfat, Natriumaluminiumchlorhydroxylactat, Aluminiumbromhydrat, Aluminiumchlorid, den Aluminiumsalzen von Lipoaminosäuren, Aluminiumsulfat, Aluminiumlactat, Aluminiumchlorhydroxyallantoinat und Natrium-Aluminium-Chlorhydroxylactat sowie Mischungen hiervon.

Erfindungsgemäß besonders bevorzugte Antitranspirant-Wirkstoffe sind ausgewählt aus so genannten "aktivierten" Aluminium- und Aluminium-Zirconiumsalzen, die auch als Antitranspirant-Wirkstoffe "mit erhöhter Wirksamkeit (englisch: enhanced activity)" bezeichnet werden. Derartige Wirkstoffe sind im Stand der Technik bekannt und auch kommerziell erhältlich. Ihre Herstellung ist beispielsweise in GB 2048229, US 4775528 und US 6010688 offenbart.

Weitere bevorzugte schweißhemmende Wirkstoffe sind basische Calcium-Aluminiumsalze, wie sie beispielsweise in US 2571030 offenbart sind. Diese Salze werden durch Umsetzen von Calciumcarbonat mit Aluminiumchlorhydroxid oder Aluminiumchlorid und Aluminiumpulver oder durch Zusetzen von Calciumchlorid-Dihydrat zu Aluminiumchlorhydroxid hergestellt.

Weitere bevorzugte schweißhemmende Wirkstoffe sind Aluminium-Zirconium-Komplexe, wie sie beispielsweise in US 4017599 offenbart sind, die mit Salzen von Aminosäuren, insbesondere mit Alkali- und Erdalkaliglycinaten, gepuffert sind.

Für die Stabilisierung der schweißhemmenden Salze bevorzugte wasserlösliche Calciumsalze sind ausgewählt aus Calciumchlorid, Calciumbromid, Calciumnitrat, Calciumcitrat, Calciumformiat, Calciumacetat, Calciumgluconat, Calciumascorbat, Calciumlactat, Calciumglycinat, Calciumcarbonat, Calciumsulfat, Calciumhydroxid, sowie Mischungen davon.

Für die Stabilisierung der schweißhemmenden Salze bevorzugte Aminosäuren sind ausgewählt aus Glycin, Alanin, Leucin, Isoleucin, β-Alanin, Valin, Cystein, Serin, Tryptophan, Phenylalanin, Methionin, β-Amino-n-butansäure und γ-Amino-n-butansäure und den Salzen davon, jeweils in der d-Form, der I-Form und der dl-Form; Glycin ist besonders bevorzugt.

Für die Stabilisierung der schweißhemmenden Salze bevorzugte Hydroxyalkansäuren sind ausgewählt aus Glycolsäure und Milchsäure.

Weitere bevorzugte schweißhemmende Wirkstoffe sind in US 6663854 und US 20040009133 offenbart.

Die schweißhemmenden Wirkstoffe liegen in gelöster Form vor. Die Antitranspirant-Wirkstoffe können als nicht-wässrige Lösungen oder als glycolische Solubilisate eingesetzt werden. Bevorzugte Aluminiumzirconiumsalze weisen ein molares Metall-zu-Chlorid-Verhältnis von 0,9 - 1,3, bevorzugt 0,9 - 1,1, besonders bevorzugt 0,9 - 1,0, auf.

Bevorzugte Aluminiumzirconiumchlorohydrate haben im allgemeinen die empirische Formel AlₙZr(OH)_{[3n+4-m(n+1)]}(Cl)_{[m(n+1)]} mit n = 2,0 - 10,0, bevorzugt 3,0 - 8,0, m = 0,77 - 1,11 (entsprechend einem molaren Metall (Al+Zr)-zu-Chlorid-Verhältnis von 1,3 - 0,9), bevorzugt m = 0,91 - 1,11 (entsprechend M:Cl = 1,1 - 0,9), und besonders bevorzugt m = 1,00 - 1,11 (entsprechend M:Cl = 1,0 - 0,9), weiterhin sehr bevorzugt m = 1,02 - 1,11 (entsprechend M:Cl = 0,98 - 0,9) sowie sehr bevorzugt m = 1,04 - 1,11 (entsprechend M:Cl = 0,96 - 0,9).

Bei diesen Salzen ist im Allgemeinen etwas Hydratationswasser assoziativ gebunden, typischerweise 1 - 6 Mol Wasser pro Mol Salz, entsprechend 1 - 16 Gew.-%, bevorzugt 4 - 13 Gew.-% Hydratationswasser.

Üblicherweise sind die bevorzugten Aluminiumzirconiumchlorohydrate mit einer Aminosäure assoziiert, um die Polymerisation der Zirconiumspecies während der Herstellung zu verhindern. Bevorzugte stabilisierende Aminosäuren sind ausgewählt aus Glycin, Alanin, Leucin, Isoleucin, β-Alanin, Cystein, Valin, Serin, Tryptophan, Phenylalanin, Methionin, β-Amino-n-butansäure und γ-Amino-n-butansäure und den Salzen davon, jeweils in der d-Form, der I-Form und der dl-Form; Glycin ist besonders bevorzugt. Die Aminosäure ist in einer Menge von 1 - 3 Mol, bevorzugt 1,3 - 1,8 Mol, jeweils pro Mol Zirconium in dem Salz enthalten.

Bevorzugte schweißhemmende Salze sind Aluminium-Zirconiumtetrachlorohydrat (Al:Zr = 2-6; M:Cl = 0.9-1.3), insbesondere Salze mit einem molaren Metall-zu-Chlorid-Verhältnis von 0,9 - 1,1, bevorzugt 0,9 - 1,0.

Weiterhin erfindungsgemäß bevorzugt sind Aluminiumzirconiumchlorohydrat-Glycin-Salze, die mit Betain ((CH₃)₃N⁺-CH₂-COO⁻) stabilisiert sind. Besonders bevorzugte entsprechende Verbindungen weisen ein molares Gesamt-(Betain + Glycin)/Zr-Verhältnis von (0,1 - 3,0): 1, bevorzugt (0,7 - 1,5) : 1 und ein molares Verhältnis von Betain zu Glycin von mindestens 0,001 : 1 auf. Entsprechende Verbindungen sind beispielsweise offenbart in US 7105691.

Weitere besonders bevorzugte Antitranspirant-Wirkstoffe sind solche Aluminiumzirconiumsalze mit einem hohen HPLC-Peak 5-Aluminium-Gehalt, die zusätzlich mit einem wasserlöslichen Strontiumsalz und/oder mit einem wasserlöslichen Calciumsalz stabilisiert sind. Entsprechende Salze sind beispielsweise in US 6923952 offenbart.

Besonders bevorzugte erfindungsgemäße Zusammensetzungen enthalten mindestens eine schweißhemmende Aluminium- oder Aluminium-Zirconium-Verbindung in einer Gesamtmenge von 5-35 Gew.-%, bevorzugt 8-30 Gew.-%, besonders bevorzugt 10-25 Gew.-% und außerordentlich bevorzugt 16 - 20 Gew.-%, jeweils bezogen auf das Gesamtgewicht der kristallwasser- und ligandenfreien Aktivsubstanz (USP) in der Gesamtzusammensetzung.

Weitere besonders bevorzugte erfindungsgemäße Zusammensetzungen enthalten mindestens eine schweißhemmende Aluminium-Verbindung in einer Gesamtmenge von 5 - 35 Gew.-%, bevorzugt 8 - 30 Gew.-%, besonders bevorzugt 10 - 25 Gew.-% und außerordentlich bevorzugt 16 - 20 Gew.-%, jeweils bezogen auf das Gesamtgewicht der kristallwasser- und ligandenfreien Aktivsubstanz (USP) in der Gesamtzusammensetzung.

Weitere besonders bevorzugte erfindungsgemäße Zusammensetzungen enthalten mindestens eine schweißhemmende Aluminium-Zirconium-Verbindung in einer Gesamtmenge von 5 - 35 Gew.-%, bevorzugt 8 - 30 Gew.-%, besonders bevorzugt 10 - 25 Gew.-% und außerordentlich bevorzugt 16 - 20 Gew.-%, jeweils bezogen auf das Gesamtgewicht der kristallwasser- und ligandenfreien Aktivsubstanz (USP) in der Gesamtzusammensetzung.

Die erfindungsgemäßen Zusammensetzungen enthalten eine Ölphase, enthaltend:
i) mindestens ein Siliconelastomer,
ii) mindestens ein unter Normalbedingungen flüssiges Siliconöl, ausgewählt aus Dimethicone und Cyclomethicone, sowie
iii) mindestens ein nur aus C und H bestehendes Kohlenwasserstofföl,
wobei kein Copolymer, bestehend aus mindestens zwei Monomeren, ausgewählt aus der Gruppe, die gebildet wird aus Ethylen, Propylen, Buten und Styren, in der Ölphase bzw. in der Zusammensetzung enthalten ist.

Überraschend wurde festgestellt, dass der Zusatz dieser Ölphase die Adhäsivität einer wässrigen Roll-on- Antitranspirant-Zusammensetzung auf einer mit der Zusammensetzung behandelten Hautoberfläche deutlich reduziert (siehe Beispielteil).

Bevorzugte erfindungsgemäße Zusammensetzungen sind dadurch gekennzeichnet, dass das mindestens eine Siliconelastomer ausgewählt ist aus Vinyl Dimethicone/Methicone Silsesquioxane Crosspolymer, Dimethicone/Vinyl Dimethicone Crosspolymer, Dimethicone/Phenyl Vinyl Dimethicone Crosspolymer, Stearyl-Vinyl/hydromethylsiloxane Copolymer, Polysilicone-11, Stearoxymethicone/Dimethicone Copolymer sowie Mischungen hiervon, besonders bevorzugt ausgewählt aus Vinyl Dimethicone/Methicone Silsesquioxane Crosspolymer.

Ein weiterer obligatorischer Bestandteil der erfindungsgemäß verwendeten Ölphase ist mindestens ein unter Normalbedingungen flüssiges Siliconöl, ausgewählt aus Dimethicone und Cyclomethicone. Unter der INCI-Bezeichnung Dimethicone werden insbesondere Polydimethylsiloxane verstanden. Erfindungsgemäß geeignet sind sowohl niedermolekulare Polydimethylsiloxane mit 2 - 10 Siloxaneinheiten, wie Hexamethyldisiloxan (L₂), Octamethyltrisiloxan (L₃), Decamethyltetrasiloxan (L₄), wie sie z.B. in den Handelsprodukten DC 2-1184, Dow Corning® 200 (0,65 cSt) und Dow Corning® 200 (1,5 cSt) von Dow Corning enthalten sind, als auch höhermolekulare Polydimethylsiloxane, die insbesondere kinematische Viskositäten (25°C) von 10 - 400 cSt aufweisen.

Unter der INCI-Bezeichnung Cyclomethicone werden insbesondere Cyclotrisiloxan (Hexamethylcyclotrisiloxan), Cyclotetrasiloxan (Octamethylcyclotetrasiloxan), Cyclopentasiloxan (Decamethylcyclopentasiloxan) und Cyclohexasiloxan (Dodecamethylcyclohexasiloxan) verstanden. Diese Öle weisen bei 20°C einen Dampfdruck von ca. 13 - 15 Pa auf. Cyclomethicone sind im Stand der Technik als gut geeignete Öle für kosmetische Produkte, insbesondere für schweißhemmende und deodorierende Produkte, bekannt. Aufgrund ihrer Persistenz in der Umwelt kann es aber erfindungsgemäß bevorzugt sein, auf den Einsatz von Cyclomethiconen zu verzichten. In einer speziell bevorzugten Ausführungsform enthalten die erfindungsgemäßen Zusammensetzungen 0 bis weniger als 1 Gew.-% Cyclomethicone, bezogen auf das Gewicht der Zusammensetzung.

Ein weiterer obligatorischer Bestandteil der erfindungsgemäß verwendeten Ölphase ist mindestens ein nur aus C und H bestehendes Kohlenwasserstofföl.

Bevorzugte erfindungsgemäße Zusammensetzungen sind dadurch gekennzeichnet, dass das mindestens eine Kohlenwasserstofföl iii) ausgewählt ist aus Mineralöl, Paraffinöl, Polyisobuten, hydriertem Polyisobuten, Polydecen, hydriertem Polydecen, C8-C20-Isoparaffinen, wie Isononan, Isodecan, Isoundecan, Isododecan, Isotridecan, Isotetradecan, Isopentadecan, Isohexadecan und Isoeicosan, sowie C10 - C20-n-Alkanen, bevorzugt C13 - C15-n-Alkanen, sowie Mischungen hiervon. Besonders bevorzugt ist hydriertes Polyisobuten.

Bevorzugte erfindungsgemäße Zusammensetzungen enthalten das mindestens eine nur aus C und H bestehende Kohlenwasserstofföl iii) in einer Gesamtmenge von 0,01 bis 10 Gew.-%, bevorzugt 0,1 bis 5 Gew.-%, besonders bevorzugt 0,5 bis 2 Gew.-%, außerordentlich bevorzugt 1,0 bis 1,5 Gew.-%, jeweils bezogen auf das Gewicht der Antitranspirant-Zusammensetzung.

Weitere bevorzugte erfindungsgemäße Zusammensetzungen enthalten das mindestens eine Siliconelastomer i) in einer Gesamtmenge von 0,01 bis 2 Gew.-%, bevorzugt 0,02 bis 1 Gew.-%, besonders bevorzugt 0,05 bis 0,2 Gew.-%, jeweils bezogen auf das Gewicht der Antitranspirant-Zusammensetzung.

Weitere bevorzugte erfindungsgemäße Zusammensetzungen enthalten mindestens ein Siliconelastomer, ausgewählt aus Dimethicone/Vinyl Dimethicone Crosspolymer, Dimethicone/Phenyl Vinyl Dimethicone Crosspolymer, Stearyl-Vinyl/hydromethylsiloxane Copolymer, Polysilicone-11, Stearoxymethicone/Dimethicone Copolymer sowie Mischungen hiervon, in einer Gesamtmenge von 0,01 bis 2,5 Gew.-%, bevorzugt 0,02 bis 2 Gew.-%, besonders bevorzugt 0,05 bis 0,5 Gew.-%, bezogen auf das Gewicht der Antitranspirant-Zusammensetzung.

Weitere bevorzugte erfindungsgemäße Zusammensetzungen enthalten das mindestens eine Siliconöl ii), ausgewählt aus Dimethicone und Cyclomethicone, in einer Gesamtmenge von 0,05 bis 5 Gew.-%, bevorzugt 0,1 bis 3 Gew.-%, besonders bevorzugt 0,8 bis 2 Gew.-%, bezogen auf das Gewicht der Antitranspirant-Zusammensetzung.

Erfindungsgemäß besonders bevorzugte Zusammensetzungen enthalten 0,5-2 Gew.-% hydriertes Polyisobuten, 0,8 - 2 Gew.-% Dimethicone und 0,05 - 0,2 Gew.-% Vinyl Dimethicone/Methicone Silsesquioxane Crosspolymer, bezogen auf das Gewicht der Antitranspirant-Zusammensetzung. Weitere erfindungsgemäß bevorzugte Zusammensetzungen enthalten 0,5 - 2 Gew.-% hydriertes Polyisobuten, 0,8 - 2 Gew.-% Cyclomethicone und 0,05 - 0,2 Gew.-% Vinyl Dimethicone/ Methicone Silsesquioxane Crosspolymer, bezogen auf das Gewicht der Antitranspirant-Zusammensetzung. Bevorzugte erfindungsgemäße Zusammensetzungen sind dadurch gekennzeichnet, dass die vorstehend beschriebene Ölphase in einer Gesamtmenge von 0,1 bis 10 Gew.-%, bevorzugt 0,5 bis 5 Gew.-%, besonders bevorzugt 1 bis 3 Gew.-%, bezogen auf das Gewicht der Antitranspirant-Zusammensetzung, enthalten ist.

Bevorzugte erfindungsgemäße Zusammensetzungen enthalten mindestens einen Öl-in-Wasser-Emulgator mit einem HLB-Wert größer 7 bis 20, der besonders bevorzugt aus nichtionischen Öl-in-Wasser-Emulgatoren mit einem HLB-Wert von größer 7 bis 20 ausgewählt ist.

Hierbei handelt es sich um dem Fachmann allgemein bekannte Emulgatoren, wie sie beispielsweise in Kirk-Othmer, "Encyclopedia of Chemical Technology", 3. Aufl., 1979, Band 8, Seiten 913 - 916, aufgelistet sind. Für ethoxylierte Produkte wird der HLB-Wert nach der Formel HLB = (100 - L) : 5 berechnet, wobei L der Gewichtsanteil der lipophilen Gruppen, das heißt der Fettalkyl- oder Fettacylgruppen, in den Ethylenoxidaddukten, ausgedrückt in Gewichtsprozent, ist.

Tenside und Emulgatoren im Sinne der vorliegenden Anmeldung sind amphiphile (bifunktionelle) Verbindungen, die aus mindestens einem hydrophoben und mindestens einem hydrophilen Molekülteil bestehen. Der hydrophobe Rest ist bevorzugt eine Kohlenwasserstoffkette mit 8-28 KohlenstoffAtomen, die gesättigt oder ungesättigt, linear oder verzweigt sein kann. Besonders bevorzugt ist diese C₈-C₂₈-Alkylkette linear. Basiseigenschaften der Tenside und Emulgatoren sind die orientierte Absorption an Grenzflächen sowie die Aggregation zu Mizellen und die Ausbildung von lyotrophen Phasen.

Weitere erfindungsgemäß bevorzugte Antitranspirant-Zusammensetzungen sind dadurch gekennzeichnet, dass mindestens ein nichtionischer Emulgator mit einem HLB-Wert im Bereich von 12 - 18 enthalten ist. Bevorzugte erfindungsgemäße Antitranspirant-Zusammensetzungen sind dadurch gekennzeichnet, dass die nichtionischen Öl-in-Wasser-Emulgatoren mit einem HLB-Wert von größer 7 bis 20 ausgewählt sind aus ethoxylierten C₈-C₂₄-Alkanolen mit durchschnittlich 10 -100 Mol Ethylenoxid pro Mol, ethoxylierten C₈-C₂₄-Carbonsäuren mit durchschnittlich 10 - 100 Mol Ethylenoxid pro Mol, mit durchschnittlich 20 - 100 Mol Ethylenoxid pro Mol ethoxylierten Sorbitanmonoestern von linearen gesättigten und ungesättigten C₁₂ - C₃₀-Carbonsäuren, die hydroxyliert sein können, insbesondere diejenigen von Myristinsäure, Palmitinsäure, Stearinsäure oder von Mischungen dieser Fettsäuren, Silicon-Copolyolen mit Ethylenoxid-Einheiten oder mit Ethylenoxid- und Propylenoxid-Einheiten, Alkylmono- und -oligoglycosiden mit 8 bis 22 Kohlenstoffatomen im Alkylrest und deren ethoxylierten Analoga, ethoxylierten Sterinen, Partialestern von Polyglycerinen mit n = 2 bis 10 Glycerineinheiten und mit 1 bis 4 gesättigten oder ungesättigten, linearen oder verzweigten, gegebenenfalls hydroxylierten C₈ - C₃₀-Fettsäureresten verestert, sofern sie einen HLB-Wert von größer 7 bis 20 aufweisen, sowie Mischungen der vorgenannten Substanzen.

Die ethoxylierten C₈-C₂₄-Alkanole haben die Formel R¹O(CH₂CH₂O)ₙH, wobei R¹ steht für einen linearen oder verzweigten Alkyl- und/oder Alkenylrest mit 8 - 24 Kohlenstoffatomen und n, die mittlere Anzahl der Ethylenoxid-Einheiten pro Molekül, für Zahlen von 10 - 100, vorzugsweise 10 - 30 Mol Ethylenoxid an 1 Mol Caprylalkohol, 2-Ethylhexylalkohol, Caprinalkohol, Laurylalkohol, Isotridecylalkohol, Myristylalkohol, Cetylalkohol, Palmitoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen. Auch Addukte von 10 - 100 Mol Ethylenoxid an technische Fettalkohole mit 12 - 18 Kohlenstoffatomen, wie beispielsweise Kokos-, Palm-, Palmkern- oder Talgfettalkohol, sind geeignet.

Die ethoxylierten C₈-C₂₄-Carbonsäuren haben die Formel R¹O(CH₂CH₂O)ₙH, wobei R¹O steht für einen linearen oder verzweigten gesättigten oder ungesättigten Acylrest mit 8 -24 Kohlenstoffatomen und n, die mittlere Anzahl der Ethylenoxid-Einheiten pro Molekül, für Zahlen von 10 - 100, vorzugsweise 10 - 30 Mol Ethylenoxid an 1 Mol Caprylsäure, 2-Ethylhexansäure, Caprinsäure, Laurinsäure, Isotridecansäure, Myristinsäure, Cetylsäure, Palmitoleinsäure, Stearinsäure, Isostearinsäure, Ölsäure, Elaidinsäure, Petroselinsäure, Arachinsäure, Gadoleinsäure, Behensäure, Erucasäure und Brassidinsäure sowie deren technische Mischungen. Auch Addukte von 10 - 100 Mol Ethylenoxid an technische Fettsäuren mit 12 - 18 Kohlenstoffatomen, wie Kokos-, Palm-, Palmkern- oder Talgfettsäure, sind geeignet. Besonders bevorzugt sind PEG-50-monostearat, PEG-100-monostearat, PEG-50-monooleat, PEG-100-monooleat, PEG-50-monolaurat und PEG-100-monolaurat.

Besonders bevorzugt eingesetzt werden die C₁₂-C₁₈-Alkanole oder die C₁₂-C₁₈-Carbonsäuren mit jeweils 10 - 30 Einheiten Ethylenoxid pro Molekül sowie Mischungen dieser Substanzen, insbesondere Ceteth-10, Ceteth-12, Ceteth-20, Ceteth-30, Steareth-10, Steareth-12, Steareth-20, Steareth-21, Steareth-30, Ceteareth-10, Ceteareth-12, Ceteareth-20, Ceteareth-30, Laureth-12 und Beheneth-20.

Bevorzugte mit durchschnittlich 20 - 100 Mol Ethylenoxid pro Mol ethoxylierte Sorbitanmonoester von linearen gesättigten und ungesättigten C₁₂ - C₃₀-Carbonsäuren, die hydroxyliert sein können, sind ausgewählt aus Polysorbate-20, Polysorbate-40, Polysorbate-60 und Polysorbate-80. Weiterhin werden vorzugsweise C₈ - C₂₂-Alkylmono- und -oligoglycoside eingesetzt. C₈ -C₂₂-Alkylmono- und -oligoglycoside stellen bekannte, handelsübliche Tenside und Emulgatoren dar. Ihre Herstellung erfolgt insbesondere durch Umsetzung von Glucose oder Oligosacchariden mit primären Alkoholen mit 8 - 22 Kohlenstoffatomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis etwa 8, vorzugsweise 1 - 2, geeignet sind. Der Oligomerisierungsgrad ist dabei ein statistischer Mittelwert, dem eine für solche technischen Produkte übliche Homologenverteilung zugrunde liegt. Produkte, die unter dem Warenzeichen Plantacare® erhältlich sind, enthalten eine glucosidisch gebundene C₈-C₁₆-Alkylgruppe an einem Oligoglucosidrest, dessen mittlerer Oligomerisationsgrad bei 1 - 2, insbesondere 1,2 - 1,4, liegt. Besonders bevorzugte C₈ - C₂₂-Alkylmono- und -oligoglycoside sind ausgewählt aus Octylglucosid, Decylglucosid, Laurylglucosid, Palmitylglucosid, Isostearylglucosid, Stearylglucosid, Arachidylglucosid und Behenylglucosid sowie Mischungen hiervon. Auch die vom Glucamin abgeleiteten Acylglucamide sind als nicht-ionische Öl-in-Wasser-Emulgatoren geeignet.

Auch ethoxylierte Sterine, insbesondere ethoxylierte Sojasterine, stellen erfindungsgemäß geeignete Öl-in-Wasser-Emulgatoren dar. Der Ethoxylierungsgrad muss größer als 5, bevorzugt mindestens 10 sein, um einen HLB-Wert größer 7 aufzuweisen. Geeignete Handelsprodukte sind z. B. PEG-10 Soy Sterol, PEG-16 Soy Sterol und PEG-25 Soy Sterol.

Weiterhin werden vorzugsweise Partialester von Polyglycerinen mit 2 bis 10 Glycerineinheiten und mit 1 bis 4 gesättigten oder ungesättigten, linearen oder verzweigten, gegebenenfalls hydroxylierten C₈ - C₃₀-Fettsäureresten verestert, eingesetzt, sofern sie einen HLB-Wert von größer 7 bis 20 aufweisen. Besonders bevorzugt sind Diglycerinmonocaprylat, Diglycerinmonocaprat, Diglycerinmonolaurat, Triglycerinmonocaprylat, Triglycerinmonocaprat, Triglycerinmonolaurat, Tetraglycerinmonocaprylat, Tetraglycerinmonocaprat, Tetraglycerinmonolaurat, Pentaglycerinmonocaprylat, Pentaglycerinmonocaprat, Pentaglycerinmonolaurat, Hexaglycerinmonocaprylat, Hexaglycerinmonocaprat, Hexaglycerinmonolaurat, Hexaglycerinmonomyristat, Hexaglycerinmonostearat, Decaglycerinmonocaprylat, Decaglycerinmonocaprat, Decaglycerinmonolaurat, Decaglycerinmonomyristat, Decaglycerinmonoisostearat, Decaglycerinmonostearat, Decaglycerinmonooleat, Decaglycerinmonohydroxystearat, Decaglycerindicaprylat, Decaglycerindicaprat, Decaglycerindilaurat, Decaglycerindimyristat, Decaglycerindüsostearat, Decaglycerindistearat, Decaglycerindioleat, Decaglycerindihydroxystearat, Decaglycerintricaprylat, Decaglycerintricaprat, Decaglycerintrilaurat, Decaglycerintrimyristat, Decaglycerintrüsostearat, Decaglycerintristearat, Decaglycerintrioleat und Decaglycerintrihydroxystearat.

Besonders bevorzugte erfindungsgemäße Antitranspirant-Zusammensetzungen enthalten mindestens einen Öl-in-Wasser-Emulgator mit einem HLB-Wert von größer 7 bis 20 in einer Gesamtmenge von 0,5 - 5 Gew.-%, bevorzugt 0,8 - 4 Gew.-%, besonders bevorzugt 1,2 - 3 Gew.-%, und außerordentlich bevorzugt 1,5 - 2 Gew.-%, jeweils bezogen auf die Gesamtzusammensetzung. Weitere erfindungsgemäß besonders bevorzugte Antitranspirant-Zusammensetzungen enthalten mindestens einen nichtionischen Öl-in-Wasser-Emulgator mit einem HLB-Wert im Bereich von 12 - 18 in einer Gesamtmenge von 0,5 - 5 Gew.-%, bevorzugt 0,8 - 4 Gew.-%, besonders bevorzugt 1,2 - 3 Gew.-%, und außerordentlich bevorzugt 1,5-2 Gew.-%, jeweils bezogen auf die Gesamtzusammensetzung.

Weitere erfindungsgemäß besonders bevorzugte Antitranspirant-Zusammensetzungen enthalten mindestens einen nichtionischen Öl-in-Wasser-Emulgator mit einem HLB-Wert im Bereich von 12 - 18, der ausgewählt ist aus linearen gesättigten und ungesättigten C₁₂ - C₂₄-Alkanolen, die mit 7 - 40 Ethylenoxid-Einheiten pro Molekül verethert sind, in einer Gesamtmenge von 0,5 - 5 Gew.-%, bevorzugt 0,8 - 4 Gew.-%, besonders bevorzugt 1,2 - 3 Gew.-%, und außerordentlich bevorzugt 1,5 - 2 Gew.-%, jeweils bezogen auf die Gesamtzusammensetzung. Besonders bevorzugt sind die vorgenannten Öl-in-Wasser-Emulgatoren ausgewählt aus Steareth, Ceteth, Myristeth, Laureth, Trideceth, Arachideth und Beheneth mit jeweils 7-40 Ethylenoxid-Einheiten pro Molekül, insbesondere Steareth-10, Steareth-20, Steareth-21, Steareth-30, Steareth-40, Ceteth-10, Ceteth-20, Ceteth-21, Ceteth-30, Ceteth-40, Laureth-10, Laureth-20, Laureth-30, Trideceth-10, Trideceth-20 und Trideceth-30, sowie Mischungen hiervon.

Weitere erfindungsgemäß bevorzugte Antitranspirant-Zusammensetzungen enthalten mindestens einen nichtionischen Öl-in-Wasser-Emulgator mit einem HLB-Wert im Bereich von 12 - 18, der ausgewählt ist aus Steareth-10, Steareth-20, Steareth-21, Steareth-30, Steareth-40, Ceteth-10, Ceteth-20, Ceteth-21, Ceteth-30, Ceteth-40, Laureth-10, Laureth-20, Laureth-30, Trideceth-10, Trideceth-20 und Trideceth-30 sowie Mischungen hiervon, in einer Gesamtmenge von 0,5 - 5 Gew.-%, bevorzugt 0,8 - 4 Gew.-%, besonders bevorzugt 1,2 - 3 Gew.-%, und außerordentlich bevorzugt 1,5 - 2 Gew.-%, jeweils bezogen auf die Gesamtzusammensetzung.

Weitere erfindungsgemäß bevorzugte Antitranspirant-Zusammensetzungen enthalten mindestens ein kosmetisches Öl und mindestens einen Öl-in-Wasser-Emulgator mit einem HLB-Wert von größer 7 bis 20 enthält und liegen als Öl-in-Wasser-Emulsion vor.

Besonders bevorzugte erfindungsgemäße Antitranspirant-Zusammensetzungen liegen als ÖI-in-Wasser-Emulsion vor und enthalten mindestens einen Öl-in-Wasser-Emulgator mit einem HLB-Wert von größer 7 bis 20 in einer Gesamtmenge von 0,5 - 5 Gew.-%, bevorzugt 0,8 - 4 Gew.-%, besonders bevorzugt 1,2 - 3 Gew.-%, und außerordentlich bevorzugt 1,5 - 2 Gew.-%, jeweils bezogen auf die Gesamtzusammensetzung.

Weitere erfindungsgemäß besonders bevorzugte Antitranspirant-Zusammensetzungen liegen als Öl-in-Wasser-Emulsion vor und enthalten mindestens einen nichtionischen Öl-in-Wasser-Emulgator mit einem HLB-Wert im Bereich von 12 - 18 in einer Gesamtmenge von 0,5 - 5 Gew.-%, bevorzugt 0,8 - 4 Gew.-%, besonders bevorzugt 1,2 - 3 Gew.-%, und außerordentlich bevorzugt 1,5 - 2 Gew.-%, jeweils bezogen auf die Gesamtzusammensetzung.

Weitere erfindungsgemäß besonders bevorzugte Antitranspirant-Zusammensetzungen liegen als Öl-in-Wasser-Emulsion vor und enthalten mindestens einen nichtionischen Öl-in-Wasser-Emulgator mit einem HLB-Wert im Bereich von 12 - 18, der ausgewählt ist aus linearen gesättigten und ungesättigten C₁₂ - C₂₄-Alkanolen, die mit 7 - 40 Ethylenoxid-Einheiten pro Molekül verethert sind, in einer Gesamtmenge von 0,5 - 5 Gew.-%, bevorzugt 0,8 - 4 Gew.-%, besonders bevorzugt 1,2 - 3 Gew.-%, und außerordentlich bevorzugt 1,5 - 2 Gew.-%, jeweils bezogen auf die Gesamtzusammensetzung. Besonders bevorzugt sind die vorgenannten Öl-in-Wasser-Emulgatoren ausgewählt aus Steareth, Ceteth, Myristeth, Laureth, Trideceth, Arachideth und Beheneth mit jeweils 7 - 40 Ethylenoxid-Einheiten pro Molekül, insbesondere Steareth-10, Steareth-20, Steareth-21, Steareth-30, Steareth-40, Ceteth-10, Ceteth-20, Ceteth-21, Ceteth-30, Ceteth-40, Laureth-10, Laureth-20, Laureth-30, Trideceth-10, Trideceth-20 und Trideceth-30, sowie Mischungen hiervon.

Weitere erfindungsgemäß bevorzugte Antitranspirant-Zusammensetzungen liegen als Öl-in-Wasser-Emulsion vor und enthalten mindestens einen nichtionischen Öl-in-Wasser-Emulgator mit einem HLB-Wert im Bereich von 12 - 18, der ausgewählt ist aus Steareth-10, Steareth-20, Steareth-21, Steareth-30, Steareth-40, Ceteth-10, Ceteth-20, Ceteth-21, Ceteth-30, Ceteth-40, Laureth-10, Laureth-20, Laureth-30, Trideceth-10, Trideceth-20 und Trideceth-30 sowie Mischungen hiervon, in einer Gesamtmenge von 0,5 - 5 Gew.-%, bevorzugt 0,8 - 4 Gew.-%, besonders bevorzugt 1,2 - 3 Gew.-%, und außerordentlich bevorzugt 1,5 - 2 Gew.-%, jeweils bezogen auf die Gesamtzusammensetzung.

### Wasser-in-Öl-Emulgatoren

Weitere erfindungsgemäß bevorzugte Antitranspirant-Zusammensetzungen enthalten mindestens einen Wasser-in-Öl-Emulgator, bevorzugt mindestens einen nichtionischen Wasser-in-Öl-Emulgator, jeweils mit einem HLB-Wert größer 1,0 und kleiner/gleich 7,0, bevorzugt im Bereich von 3 - 6. Einige dieser Wasser-in-Öl-Emulgatoren sind beispielsweise in Kirk-Othmer, "Encyclopedia of Chemical Technology", 3. Aufl., 1979, Band 8, Seite 913, aufgelistet. Für ethoxylierte Addukte lässt sich der HLB-Wert, wie bereits erwähnt, auch berechnen.

Als Wasser-in-Öl-Emulgator bevorzugt sind:
- lineare oder verzweigte, gesättigte oder ungesättigte C₁₂ - C₃₀-Alkanole, die jeweils mit 1 - 4 Ethylenoxid-Einheiten pro Molekül verethert sind, welche außerordentlich bevorzugt sind aus Steareth, Ceteth, Myristeth, Laureth, Trideceth, Arachideth und Beheneth mit jeweils 1 - 4 Ethylenoxid-Einheiten pro Molekül, insbesondere Steareth-2, Steareth-3, Steareth-4, Ceteth-2, Ceteth-3, Ceteth-4, Myristeth-2, Myristeth-3, Myristeth-4, Laureth-2, Laureth-3, Laureth-4, Trideceth-2, Trideceth-3 und Trideceth-4 sowie Mischungen hiervon;
- lineare gesättigte Alkanole mit 12 - 30 Kohlenstoffatomen, insbesondere mit 16 - 22 Kohlenstoffatomen, insbesondere Cetylalkohol, Stearylalkohol, Arachidylalkohol, Behenylalkohol und Lanolinalkohol oder Gemische dieser Alkohole, wie sie bei der technischen Hydrierung von pflanzlichen und tierischen Fettsäuren erhältlich sind;
- Ester und insbesondere Partialester aus einem Polyol mit 2 - 6 C-Atomen und linearen gesättigten und ungesättigten Fettsäuren mit 12 - 30, insbesondere 14 - 22 C-Atomen, die hydroxyliert sein können. Solche Ester oder Partialester sind z. B. die Mono- und Diester von Glycerin oder Ethylenglycol oder die Monoester von Propylenglycol mit linearen gesättigten und ungesättigten C₁₂ - C₃₀-Carbonsäuren, die hydroxyliert sein können, insbesondere diejenigen mit Palmitin- und Stearinsäure, die Sorbitanmono-, -di- oder -triester von linearen gesättigten und ungesättigten C₁₂ - C₃₀-Carbonsäuren, die hydroxyliert sein können, insbesondere diejenigen von Myristinsäure, Palmitinsäure, Stearinsäure oder von Mischungen dieser Fettsäuren, die Pentaerythritylmono-, -di-, -tri- und -tetraester und die Methylglucosemono- und -diester von linearen, gesättigten und ungesättigten C₁₂ - C₃₀-Carbonsäuren, die hydroxyliert sein können, wovon besonders bevorzugt sind die Mono-, Di-, Tri- und Tetraester von Pentaerythrit mit linearen gesättigten Fettsäuren mit 12-30, insbesondere 14-22 Kohlenstoffatomen, die hydroxyliert sein können, sowie Mischungen hiervon. Erfindungsgemäß besonders bevorzugt sind die Mono- und Diester. Erfindungsgemäß bevorzugte C₁₂-C₃₀-Fettsäurereste sind ausgewählt aus Laurinsäure-, Myristinsäure-, Palmitinsäure-, Stearinsäure-, Arachinsäure- und Behensäure-Resten; besonders bevorzugt ist der Stearinsäurerest. Erfindungsgemäß besonders bevorzugte nichtionische Wasser-in-Öl-Emulgatoren mit einem HLB-Wert größer 1,0 und kleiner/gleich 7,0 sind ausgewählt aus Glycerylmonostearat, Glyceryldistearat, Glycerylmonopalmitat, Glyceryldipalmitat und Mischungen hiervon;
- Sterine, also Steroide, die am C3-Atom des Steroid-Gerüstes eine Hydroxylgruppe tragen und sowohl aus tierischem Gewebe (Zoosterine, z. B. Cholesterin, Lanosterin) wie auch aus Pflanzen (Phytosterine, z.B. Ergosterin, Stigmasterin, Sitosterin) und aus Pilzen und Hefen (Mykosterine) isoliert werden und die niedrig ethoxyliert (1 - 5 EO) sein können;
- Alkanole und Carbonsäuren mit jeweils 8 - 24 C-Atomen, insbesondere mit 16 - 22 C-Atomen, in der Alkylgruppe und 1 - 4 Ethylenoxid-Einheiten pro Molekül, die einen HLB-Wert größer 1,0 und kleiner/gleich 7,0 aufweisen,
- Glycerinmonoether gesättigter und/oder ungesättigter, verzweigter und/oder unverzweigter Alkohole einer Kettenlänge von 8 - 30, insbesondere 12 - 18 Kohlenstoffatomen,
- Partialester von Polyglycerinen mit n = 2 bis 10 Glycerineinheiten und mit 1 bis 5 gesättigten oder ungesättigten, linearen oder verzweigten, gegebenenfalls hydroxylierten C₈ - C₃₀-Fettsäureresten verestert, sofern sie einen HLB-Wert größer 1,0 bis kleiner/gleich als 7 aufweisen,
- sowie Mischungen der vorgenannten Substanzen.

Besonders bevorzugt ist der mindestens eine Wasser-in-Öl-Emulgator mit einem HLB-Wert größer 1,0 und kleiner/gleich 7,0, bevorzugt im Bereich von 3 - 6, ausgewählt aus linearen oder verzweigten, gesättigten oder ungesättigten C₁₂ - C₃₀-Alkanole, die jeweils mit 1 - 4 Ethylenoxid-Einheiten pro Molekül verethert sind, welche außerordentlich bevorzugt sind aus Steareth, Ceteth, Myristeth, Laureth, Trideceth, Arachideth und Beheneth mit jeweils 1 - 4 Ethylenoxid-Einheiten pro Molekül, insbesondere Steareth-2, Steareth-3, Steareth-4, Ceteth-2, Ceteth-3, Ceteth-4, Myristeth-2, Myristeth-3, Myristeth-4, Laureth-2, Laureth-3, Laureth-4, Trideceth-2, Trideceth-3 und Trideceth-4 sowie Mischungen hiervon.

Erfindungsgemäß kann es bevorzugt sein, nur einen einzigen Wasser-in-Öl-Emulgator einzusetzen. In einer anderen bevorzugten Ausführungsform enthalten die erfindungsgemäßen Zusammensetzungen Mischungen, insbesondere technische Mischungen, von mindestens zwei Wasser-in-ÖI-Emulgatoren.

Erfindungsgemäß bevorzugte Antitranspirant-Zusammensetzungen enthalten mindestens einen Wasser-in-Öl-Emulgator mit einem HLB-Wert größer 1,0 und kleiner/gleich 7,0, bevorzugt im Bereich von 3 - 6, in einer Gesamtmenge von 1,8 - 3 Gew.-%, bevorzugt 2 - 2,8 Gew.-% und besonders bevorzugt 2,4 - 2,6 Gew.-%, jeweils bezogen auf das Gesamtgewicht der erfindungsgemäßen Zusammensetzung.

Weitere erfindungsgemäß bevorzugte Antitranspirant-Zusammensetzungen enthalten mindestens einen nichtionischen Wasser-in-Öl-Emulgator mit einem HLB-Wert im Bereich von 3 - 6, ausgewählt aus Steareth-2, Steareth-3, Steareth-4, Ceteth-2, Ceteth-3, Ceteth-4, Myristeth-2, Myristeth-3, Myristeth-4, Laureth-2, Laureth-3, Laureth-4, Trideceth-2, Trideceth-3 und Trideceth-4 sowie Mischungen hiervon, in einer Gesamtmenge von 1,8 - 3 Gew.-%, bevorzugt 2 - 2,8 Gew.-% und besonders bevorzugt 2,4 - 2,6 Gew.-%, jeweils bezogen auf das Gesamtgewicht der erfindungsgemäßen Zusammensetzung.

Weitere erfindungsgemäß bevorzugte Antitranspirant-Zusammensetzungen liegen als Öl-in-Wasser-Emulsion vor und enthalten mindestens einen Wasser-in-Öl-Emulgator mit einem HLB-Wert größer 1,0 und kleiner/gleich 7,0, bevorzugt im Bereich von 3 - 6, in einer Gesamtmenge von 1,8 - 3 Gew.-%, bevorzugt 2 - 2,8 Gew.-% und besonders bevorzugt 2,4 - 2,6 Gew.-%, jeweils bezogen auf das Gesamtgewicht der erfindungsgemäßen Zusammensetzung.

Weitere erfindungsgemäß bevorzugte Antitranspirant-Zusammensetzungen liegen als Öl-in-Wasser-Emulsion vor und enthalten mindestens einen nichtionischen Wasser-in-Öl-Emulgator mit einem HLB-Wert im Bereich von 3 - 6, ausgewählt aus Steareth-2, Steareth-3, Steareth-4, Ceteth-2, Ceteth-3, Ceteth-4, Myristeth-2, Myristeth-3, Myristeth-4, Laureth-2, Laureth-3, Laureth-4, Trideceth-2, Trideceth-3 und Trideceth-4 sowie Mischungen hiervon, in einer Gesamtmenge von 1,8 - 3 Gew.-%, bevorzugt 2 - 2,8 Gew.-% und besonders bevorzugt 2,4 - 2,6 Gew.-%, jeweils bezogen auf das Gesamtgewicht der erfindungsgemäßen Zusammensetzung.

### Ethanol

Weitere erfindungsgemäß bevorzugte Antitranspirant-Zusammensetzungen sind dadurch gekennzeichnet, dass Ethanol in einer Menge von 0 bis 5 Gew.-%, bevorzugt 0 bis 3 Gew.-%, besonders bevorzugt 0 bis 1 Gew.-%, jeweils bezogen auf das Gewicht der Zusammensetzung, enthalten ist. Erfindungsgemäß bevorzugte Antitranspirant-Zusammensetzungen liegen in Form einer Öl-in-Wasser-Emulsion vor.

### Öle

Erfindungsgemäß bevorzugte Antitranspirant-Zusammensetzungen enthalten zusätzlich zu dem mindestens einen Siliconöl c) ii), ausgewählt aus Dimethicone und Cyclomethicone, und zusätzlich zu dem mindestens einen Kohlenwasserstofföl c) iii) mindestens ein weiteres kosmetisches Öl, bevorzugt in einer Gesamtmenge von 0,1 - 5 Gew.-%, besonders bevorzugt 0,3 - 3 Gew.-%, außerordentlich bevorzugt 0,5 - 1 Gew.-%, bezogen auf das Gewicht der Antitranspirant-Zusammensetzung.

Bei den kosmetischen Ölen unterscheidet man flüchtige und nicht-flüchtige Öle. Unter nicht-flüchtigen Ölen versteht man solche Öle, die bei 20 °C und einem Umgebungsdruck von 1013 hPa einen Dampfdruck von weniger als 2,66 Pa (0,02 mm Hg) aufweisen. Unter flüchtigen Ölen versteht man solche Öle, die bei 20 °C und einem Umgebungsdruck von 1013 hPa einen Dampfdruck von 2,66 Pa - 40000 Pa (0,02 mm - 300 mm Hg), bevorzugt 13 - 12000 Pa (0,1 - 90 mm Hg), besonders bevorzugt 15 - 3000 Pa, außerordentlich bevorzugt 30 - 500 Pa, aufweisen.

Erfindungsgemäß besonders bevorzugte nichtflüchtige Nichtsiliconöle sind ausgewählt aus den Anlagerungsprodukten von mindestens 6 Ethylenoxid- und/oder Propylenoxid-Einheiten an ein- oder mehrwertige C₃₋₂₂-Alkanole wie Butanol, Butandiol, Myristylalkohol und Stearylalkohol, z.B. PPG-13-Butylether, PPG-14-Butylether, PPG-9-Butylether, PPG-10-Butandiol, PPG-15-Stearylether sowie Mischungen hiervon.

Erfindungsgemäß besonders bevorzugte Zusammensetzungen enthalten mindestens ein kosmetisches Öl, ausgewählt aus PPG-13-Butylether, PPG-14-Butylether, PPG-9-Butylether, PPG-10-Butandiol, PPG-15-Stearylether sowie Mischungen hiervon, in einer Gesamtmenge von 0,1 - 15 Gew.-%, besonders bevorzugt 0,3 - 10 Gew.-%, außerordentlich bevorzugt 0,5 - 6 Gew.-%, bezogen auf das Gewicht der gesamten erfindungsgemäßen Antitranspirant-Zusammensetzung. Außerordentlich bevorzugte erfindungsgemäße Zusammensetzungen enthalten 0,1 - 15 Gew.-%, besonders bevorzugt 0,3 - 10 Gew.-%, außerordentlich bevorzugt 0,5 - 6 Gew.-%, PPG-15-Stearylether, jeweils bezogen auf das Gewicht der gesamten erfindungsgemäßen Antitranspirant-Zusammensetzung.

Weitere erfindungsgemäß besonders bevorzugte nichtflüchtige Nichtsiliconöle sind Ester der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können. Bevorzugt sind Ester der linearen oder verzweigten gesättigten Fettalkohole mit 2 - 5 Kohlenstoffatomen mit linearen oder verzweigten gesättigten oder ungesättigten Fettsäuren mit 10 - 18 Kohlenstoffatomen, die hydroxyliert sein können. Bevorzugte Beispiele hierfür sind Isopropylpalmitat, Isopropylstearat, Isopropylmyristat, 2-Hexyldecylstearat, 2-Hexyldecyllaurat, Isononylisononanoat, 2-Ethylhexylpalmitat und 2-Ethylhexylstearat. Ebenfalls bevorzugt sind Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Düsotridecylacetat, n-Hexyllaurat, n-Decyloleat, Oleyloleat, Oleylerucat, Erucyloleat, C₁₂-C₁₅-Alkyllactat und Di-C₁₂-C₁₃-Alkylmalat sowie die Benzoesäureester von linearen oder verzweigten C₈-₂₂-Alkanolen. Besonders bevorzugt sind Benzoesäure-C₁₂-C₁₅-Alkylester, z. B. erhältlich als Handelsprodukt Finsolv® TN (C₁₂-C₁₅-Alkylbenzoat), sowie Benzoesäureisostearylester, z.B. erhältlich als Finsolv® SB, 2-Ethylhexylbenzoat, z. B. erhältlich als Finsolv® EB, und Benzoesäure-2-octyldodecylester, z. B. erhältlich als Finsolv® BOD. Ein weiteres besonders bevorzugtes Esteröl ist Triethylcitrat.

Weitere erfindungsgemäß bevorzugte nichtflüchtige Nichtsiliconöle sind ausgewählt aus verzweigten gesättigten oder ungesättigten Fettalkoholen mit 6 - 30 Kohlenstoffatomen. Diese Alkohole werden häufig auch als Guerbet-Alkohole bezeichnet, da sie nach der Guerbet-Reaktion erhältlich sind. Bevorzugte Alkoholöle sind 2-Hexyldecanol, 2-Octyldodecanol und 2-Ethylhexylalkohol. Ebenfalls bevorzugt ist Isostearylalkohol. Weitere bevorzugte nichtflüchtige Öle sind ausgewählt aus Mischungen aus Guerbetalkoholen und Guerbetalkoholestern, z. B. 2-Hexyldecanol und 2-Hexyldecyllaurat.

Der im Folgenden gebrauchte Ausdruck "Triglycerid" meint "Glycerintriester". Weitere erfindungsgemäß bevorzugte nichtflüchtige Öle sind ausgewählt aus den Triglyceriden von linearen oder verzweigten, gesättigten oder ungesättigten, gegebenenfalls hydroxylierten C₈₋₃₀-Fettsäuren, sofern diese unter Normalbedingungen flüssig sind. Besonders geeignet kann die Verwendung natürlicher Öle, z.B. Sojaöl, Baumwollsaatöl, Sonnenblumenöl, Palmöl, Palmkernöl, Leinöl, Mandelöl, Rizinusöl, Maisöl, Rapsöl, Olivenöl, Sesamöl, Distelöl, Weizenkeimöl, Pfirsichkernöl und die flüssigen Anteile des Kokosöls und dergleichen sein. Besonders bevorzugt sind synthetische Triglyceridöle, insbesondere Capric/Caprylic Triglycerides, z. B. die Handelsprodukte Myritol® 318 oder Myritol® 331 (BASF) mit unverzweigten Fettsäureresten sowie Glyceryltrüsostearin und Glyceryltri(2-ethylhexanoat) mit verzweigten Fettsäureresten. Derartige Triglyceridöle machen bevorzugt einen Anteil von weniger als 50 Gew.-% am Gesamtgewicht aller kosmetischen Öle in der erfindungsgemäßen Zusammensetzung aus.

Weitere erfindungsgemäß besonders bevorzugte nichtflüchtige Nichtsiliconöle sind ausgewählt aus den Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Di-n-butyl/Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat.

Weitere erfindungsgemäß besonders bevorzugte nichtflüchtige Nichtsiliconöle sind ausgewählt aus den Anlagerungsprodukten von 1 bis 5 Propylenoxid-Einheiten an ein- oder mehrwertige C₈₋₂₂-Alkanole wie Octanol, Decanol, Decandiol, Laurylalkohol, Myristylalkohol und Stearylalkohol, bevorzugt aus PPG-2-Myristylether und PPG-3-Myristylether.

Weitere erfindungsgemäß besonders bevorzugte nichtflüchtige Nichtsiliconöle sind ausgewählt aus den symmetrischen, unsymmetrischen oder cyclischen Estern der Kohlensäure mit C₆₋C₂₀-Alkoholen, z. B. Di-n-caprylylcarbonat oder Di-(2-ethylhexyl)carbonat. Ester der Kohlensäure mit C₁-C₅-Alkoholen, z. B. Glycerincarbonat oder Propylencarbonat, sind hingegen keine als kosmetisches Öl geeigneten Verbindungen.

Weitere Öle, die erfindungsgemäß bevorzugt sein können, sind ausgewählt aus den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen. Besonders bevorzugt beträgt das Gesamtgewicht an Dimerfettsäureestern 0,5 - 10 Gew.-%, bevorzugt 1 - 5 Gew.-%, jeweils bezogen auf die gesamte Zusammensetzung.

Weitere erfindungsgemäß bevorzugte Antitranspirant-Zusammensetzungen in Form von Öl-in-Wasser-Emulsionen sind dadurch gekennzeichnet, dass der mindestens eine Propylenglycolmonoester von verzweigten gesättigten C₆ - C₃₀-Alkancarbonsäuren ausgewählt ist aus Propylenglycolmonoisostearat, Propylenglycolmonoisopalmitat, Propylenglycolmonoisobehenat, Propylenglycolmonoisoarachinat, Propylenglycolmonoisomyristat, Propylenglycolmonoisocaprat, Propylenglycolmonoisocaprinat und Propylenglycolmonoisocaprylat sowie Mischungen hiervon. Weitere erfindungsgemäß bevorzugte Antitranspirant-Zusammensetzungen in Form von Öl-in-Wasser-Emulsionen sind dadurch gekennzeichnet, dass das mindestens eine verzweigte gesättigte C₁₀ - C₃₀-Alkanol ausgewählt ist aus Isostearylalkohol, Isocetylalkohol, Isomyristylalkohol, Isotridecylalkohol, Isoarachidylalkohol, Isobehenylalkohol, Isocaprylalkohol, Isocaprinylalkohol, Isocaprylylalkohol, sowie Mischungen hiervon.

Weitere erfindungsgemäß bevorzugte Antitranspirant-Zusammensetzungen in Form von Öl-in-Wasser-Emulsionen sind dadurch gekennzeichnet, dass mindestens ein nichtionischer Emulgator mit einem HLB-Wert im Bereich von 3 - 6 und mindestens ein nichtionischer Emulgator mit einem HLB-Wert im Bereich von 12 - 18 enthalten sind.

Weitere erfindungsgemäß bevorzugte Antitranspirant-Zusammensetzungen in Form von Öl-in-Wasser-Emulsionen sind dadurch gekennzeichnet, dass mindestens ein nichtionischer Emulgator mit einem HLB-Wert im Bereich von 3 - 6 in einer Gesamtmenge von 1,8 - 3 Gew.-% und mindestens ein nichtionischer Emulgator mit einem HLB-Wert im Bereich von 12 - 18 in einer Gesamtmenge von 1 - 2 Gew.-%, enthalten sind, wobei die Mengenangaben jeweils auf das Gesamtgewicht der erfindungsgemäßen Zusammensetzung bezogen sind.

Weitere erfindungsgemäß bevorzugte Antitranspirant-Zusammensetzungen in Form von Öl-in-Wasser-Emulsionen sind dadurch gekennzeichnet, dass als nichtionischer Emulgator mit einem HLB-Wert im Bereich von 3 - 6 Steareth-2 und gleichzeitig als nichtionischer Emulgator mit einem HLB-Wert im Bereich von 12 - 18 Steareth-21 enthalten ist.

Weitere erfindungsgemäß bevorzugte Antitranspirant-Zusammensetzungen in Form von Öl-in-Wasser-Emulsionen sind dadurch gekennzeichnet, dass Steareth-2, Steareth-21 und PPG-15-Stearylether enthalten sind.

### Zusatzstoffe

Zusätzlich zu den vorgenannten Inhaltsstoffen können die erfindungsgemäßen Zusammensetzungen weitere Zusatz- und Hilfsstoffe enthalten, die beispielsweise ihre Haltbarkeit verbessern, wie Konservierungsmittel, z. B. Phenoxyethanol, Methylparaben oder Propylparaben, Antioxidantien, z. B. Tetradibutyl Pentaerythrityl Hydroxyhydrocinnamate, Lipochroman-6, Tocopherol, Tocopherylacetat oder Ascorbinsäure und deren Derivate, Vitamine und deren Derivate, wie Tocopherol, Tocopherylacetat, Ascorbinsäure, Panthenol oder Pantolacton, Parfüms, etherische Öle, Menthol und Mentholderivate, die eine hautkühlende Wirkung zeigen, Pflegestoffe mit Haut beruhigender Wirkung, wie Bisabolol und Allantoin, Wirkstoffe, die das Haarwachstum verzögern, z. B. Eflornithin oder Glycyrrhizin und dessen Derivate, Moisturizer und Feuchthaltemittel, wie 1,2-Propylenglycol, Glycerin, 2-Methyl-1,3-propandiol, 1,2-Butylenglycol, 1,3-Butylenglycol, 1,4-Butylenglycol, Pentylenglycole wie 1,2-Pentandiol und 1,5-Pentandiol, Hexandiole wie 1,2-Hexandiol und 1,6-Hexandiol, Hexantriole, wie 1,2,6-Hexantriol, 1,2-Octandiol, 1,8-Octandiol, Dipropylenglycol, Tripropylenglycol, Diglycerin, Triglycerin, Erythrit, Sorbit, cis-1,4-Dimethylolcyclohexan, trans-1,4-Dimethylolcyclohexan, beliebige Isomeren-Gemische von cis- und trans-1,4-Dimethylolcyclohexan, Harnstoff, N,N'-Bis-(2-Hydroxyethyl)harnstoff, Natriumpyrrolidoncarboxylat, Pflanzenextrakte, z. B. Aloe vera-Extrakt, natürliche Fette und Öle, wie Jojobaöl, Nachtkerzenöl oder Leinöl, gesättigte und ungesättigte Fettsäuren, wie Stearinsäure, Ölsäure, Linolsäure, Linolensäure oder gamma-Linolensäure, Squalan, Squalen, Deodorantwirkstoffe, wie Silbersalze, kolloidales Silber, Zeolithe, 2-Benzylheptan-1-ol, Anisalkohol, Mischungen von 2-Benzylheptan-1-ol und Phenoxyethanol, 3-(2-Ethylhexyloxy)-1,2-propandiol oder Tropolon, Verdickungsmittel, wie Dehydroxanthan Gum, Cellulosen, Celluloseether und Stärkederivate sowie Mischungen dieser Stoffe.

Bevorzugte erfindungsgemäße Zusammensetzungen weisen eine Viskosität im Bereich von 1000 bis 10000 mPas, bevorzugt 1500 bis 3000 mPas, auf, wobei die Viskosität bei 23°C mit einem Rotationsviskosimeter der Firma Brookfield, Gerät RVF, Spindel 4, Scherrate (Umdrehungsfrequenz) 20 min⁻¹, ohne Helipath, gemessen wird.

Ein weiterer Gegenstand der vorliegenden Anmeldung ist ein nicht-therapeutisches Verfahren zur schweißhemmenden Behandlung der Haut, das dadurch gekennzeichnet ist, dass eine erfindungsgemäße oder erfindungsgemäß bevorzugte Antitranspirant-Zusammensetzung auf die Haut appliziert wird.

Bezüglich weiterer bevorzugter Ausführungsformen des erfindungsgemäßen Verfahrens gilt mutatis mutandis das zu den erfindungsgemäßen und erfindungsgemäß bevorzugten Antitranspirant-Zusammensetzungen Gesagte.

Ein weiterer Gegenstand der vorliegenden Anmeldung ist die Verwendung einer Mischung, bestehend aus
i) mindestens einem Siliconelastomer,
ii) mindestens einem unter Normalbedingungen flüssiges Siliconöl, ausgewählt aus Dimethicone und Cyclomethicone, sowie
iii) mindestens einem nur aus C und H bestehenden Kohlenwasserstofföl,
zur Reduzierung der Adhäsivität einer Antitranspirant-Zusammensetzung zur Roll-on-Applikation auf die Haut, enthaltend Wasser und mindestens eine schweißhemmende Aluminium- oder Aluminium-Zirconium-Verbindung, auf der Haut, wobei die Antitranspirant-Zusammensetzung kein Copolymer, bestehend aus mindestens zwei Monomeren, ausgewählt aus der Gruppe, die gebildet wird aus Ethylen, Propylen, Buten und Styren, enthält.

Bezüglich weiterer bevorzugter Ausführungsformen der erfindungsgemäßen Verwendung gilt mutatis mutandis das zu den erfindungsgemäßen und erfindungsgemäß bevorzugten Antitranspirant-Zusammensetzungen Gesagte.

Ein weiterer Gegenstand der vorliegenden Anmeldung ist ein Verfahren zur Herstellung einer Antitranspirant-Zusammensetzung zur Roll-on-Applikation auf die Haut mit reduzierter Adhäsivität auf der Haut, wobei eine Mischung, bestehend aus
i) mindestens einem Siliconelastomer,
ii) mindestens einem unter Normalbedingungen flüssiges Siliconöl, ausgewählt aus Dimethicone und Cyclomethicone, sowie
iii) mindestens einem nur aus C und H bestehenden Kohlenwasserstofföl,
mit Wasser und mindestens einer schweißhemmenden Aluminium- oder Aluminium-Zirconium-Verbindung vermischt wird.

Bezüglich weiterer bevorzugter Ausführungsformen des erfindungsgemäßen Herstellverfahrens gilt mutatis mutandis das zu den erfindungsgemäßen und erfindungsgemäß bevorzugten Antitranspirant-Zusammensetzungen Gesagte.

Die folgenden Ausführungsbeispiele sollen den Gegenstand der vorliegenden Erfindung verdeutlichen, ohne ihn hierauf zu beschränken.

### Beispiele:

### Beispiel 1: Experimentelle Bestimmung der Adhäsivität

Folgende Roll on-Emulsionen vom Typ Öl-in-Wasser wurden hergestellt:

| | A | B | C | D |
|---|---|---|---|---|
| | Gew.-% | Gew.-% | Gew.-% | Gew.-% |
| PPG-15 Stearyl Ether* | 0,5 | 0,5 | 0,5 | 0,5 |
| Steareth-2 | 2,4 | 2,4 | 2,4 | 2,4 |
| Steareth-21 | 1,5 | 1,5 | 1,5 | 1,5 |
| Aluminium Chlorohydrate | 10 | 10 | 10 | 10 |
| Hydrogenated Polyisobutene* | - | 1 | - | 1 |
| Dimethicone (86 Gew.-%) (and) Dimethicone Crosspolymer (14 Gew.-%)* ** | - | - | 1 | 1 |
| Wasser | ad 100 | ad 100 | ad 100 | ad 100 |

| | | | | |
|---|---|---|---|---|
| * Bestandteile der Ölphase ** Rohstoff Dow Corning 9041 | | | | |

Die Adhäsivität unterschiedlicher Formulierungen wurde durch Kraftmessung mit TA.XT Express (Stable Microsystems) ermittelt. Dabei wurden die Formulierungen auf einen 6x6 cm großen und 200g schweren, mit Kollagen beschichteten Probenkörper aufgetragen. Der Probenkörper wurde dann über eine Strecke von 60 mm mit einer Geschwindigkeit von 5 mm pro Sekunde über eine mit Kollagen beschichtete Oberfläche bewegt. Die hierzu erforderliche Kraft wurde gemessen und in µN angegeben. Die Daten in Tabelle 1 zeigen, dass die Adhäsivität der Formulierung A sowohl durch Zusatz von Hydrogenated Polyisobutene (Formulierung B) als auch von Dimethicone (and) Dimethicone Crosspolymer (Formulierung C) reduziert wird. Durch die Kombination würde sich rechnerisch ein additiver Effekt von -14,5 µN gegenüber der Formulierung A ergeben. Der experimentelle Wert der Kombinationsformel D liegt jedoch bei -17,8 µN und zeigt eine synergistische Wirkung der beiden Komponenten.

**Tabelle 1: Adhäsivität unterschiedlicher Formulierungen**

| | A | B | C | D |
|---|---|---|---|---|
| Adhäsivität (µN) | 43,4 | 40,2 | 32,2 | 25,6 |
| Differenz zu A (µN) | - | -3,2 | -11,2 | -17,8 |

Außerdem wurden die Formulierungen A-D als Roll-On in der Achsel angewendet. Auch hier zeigt sich die Formulierung D als deutlich weniger klebrig im Vergleich zu den Formulierungen A-C. Dies bestätigt das Ergebnis der physikalischen Adhäsivitätsmessung (Tabelle 1).

### Beispiel 2: Antitranspirant Roll-On

| | 1 | 2 | 3 | 4 | 5 |
|---|---|---|---|---|---|
| | Gew.-% | Gew.-% | Gew.-% | Gew.-% | Gew.-% |
| PPG-15 Stearyl Ether* | 0,5 | 0,5 | 0,5 | 0,5 | 0,5 |
| Steareth-2 | 2,4 | 2,4 | 2,4 | 2,4 | 2,4 |
| Steareth-21 | 1,5 | 1,5 | 1,5 | 1,5 | 1,5 |
| Aluminium Chlorohydrate | 10 | 15 | 20 | - | - |
| Aluminium Sesquichlorohydrate (Reach 301L, 40% in Wasser) | - | - | - | 25 | - |
| Aluminium Zirconium Tetrachlorohydrex Gly | - | - | - | - | 20 |
| Hydrogenated Polyisobutene* | 1 | 1 | 1 | 1 | 1 |
| Dimethicone (86 Gew.-%) (and) Dimethicone Crosspolymer (14 Gew.-%)* ** | 1 | 1 | 1 | 1 | 1 |
| Wasser | ad 100 | ad 100 | ad 100 | ad 100 | ad 100 |

| | | | | | |
|---|---|---|---|---|---|
| * Bestandteile der Ölphase ** Rohstoff Dow Corning 9041 | | | | | |

## Patentansprüche

1. Antitranspirant-Zusammensetzung zur Roll-on-Applikation, enthaltend:
a. Wasser,
b. mindestens eine schweißhemmende Aluminium- oder Aluminium-Zirconium-Verbindung,
c. eine Ölphase, enthaltend:
i. mindestens ein Siliconelastomer,
ii. mindestens ein unter Normalbedingungen flüssiges Siliconöl, ausgewählt aus Dimethicone und Cyclomethicone, sowie
iii. mindestens ein nur aus C und H bestehendes Kohlenwasserstofföl,
wobei die Zusammensetzung kein Copolymer, bestehend aus mindestens zwei Monomeren, ausgewählt aus der Gruppe, die gebildet wird aus Ethylen, Propylen, Buten und Styren, enthält.

2. Zusammensetzung nach Anspruch 1, **dadurch gekennzeichnet, dass** das mindestens eine Siliconelastomer i) ausgewählt ist aus Vinyl Dimethicone/Methicone Silsesquioxane Crosspolymer, Dimethicone/Vinyl Dimethicone Crosspolymer, Dimethicone/Phenyl Vinyl Dimethicone Crosspolymer, Stearyl-Vinyl/hydromethylsiloxane Copolymer, Polysilicone-11, Stearoxymethicone/Dimethicone Copolymer sowie Mischungen hiervon, bevorzugt ausgewählt aus Vinyl Dimethicone/Methicone Silsesquioxane Crosspolymer.

3. Zusammensetzung nach einem der Ansprüche 1 oder 2, **dadurch gekennzeichnet, dass** das mindestens eine Kohlenwasserstofföl iii) ausgewählt ist aus Mineralöl, Paraffinöl, Polyisobuten, hydriertem Polyisobuten, Polydecen, hydriertem Polydecen, C8-C20-Isoparaffinen, wie Isononan, Isodecan, Isoundecan, Isododecan, Isotridecan, Isotetradecan, Isopentadecan, Isohexadecan und Isoeicosan, sowie C10 - C20-n-Alkanen, bevorzugt C13 - C15-n-Alkanen, sowie Mischungen hiervon.

4. Zusammensetzung nach einem der Ansprüche 1 bis 3, **dadurch gekennzeichnet, dass** das mindestens eine Siliconelastomer i) in einer Gesamtmenge von 0,01 bis 2 Gew.-%, bevorzugt 0,02 bis 1 Gew.-%, besonders bevorzugt 0,05 bis 0,2 Gew.-%, jeweils bezogen auf das Gewicht der Antitranspirant-Zusammensetzung, enthalten ist.

5. Zusammensetzung nach einem der Ansprüche 1 bis 4, **dadurch gekennzeichnet, dass** das mindestens eine Siliconöl ii), ausgewählt aus Dimethicone und Cyclomethicone, in einer Gesamtmenge von 0,05 bis 5 Gew.-%, bevorzugt 0,1 bis 3 Gew.-%, besonders bevorzugt 0,8 bis 2 Gew.-%, bezogen auf das Gewicht der Antitranspirant-Zusammensetzung, enthalten ist.

6. Zusammensetzung nach einem der Ansprüche 1 bis 5, **dadurch gekennzeichnet, dass** das mindestens eine nur aus C und H bestehende Kohlenwasserstofföl iii) in einer Gesamtmenge von 0,01 bis 10 Gew.-%, bevorzugt 0,1 bis 5 Gew.-%, besonders bevorzugt 0,5 bis 2 Gew.-%, außerordentlich bevorzugt 1,0 bis 1,5 Gew.-%, jeweils bezogen auf das Gewicht der Antitranspirant-Zusammensetzung, enthalten ist.

7. Zusammensetzung nach einem der Ansprüche 1 bis 6, **dadurch gekennzeichnet, dass** mindestens ein ÖI-in-Wasser-Emulgator mit einem HLB-Wert von größer 7 bis 20, bevorzugt mindestens ein nichtionischer ÖI-in-Wasser-Emulgator mit einem HLB-Wert von größer 7 bis 20, und gegebenenfalls mindestens ein Wasser-in-ÖI-Emulgator mit einem HLB-Wert größer 1,0 und kleiner/gleich 7,0, bevorzugt im Bereich von 3 - 6, enthalten ist.

8. Zusammensetzung nach einem der Ansprüche 1 bis 7, **dadurch gekennzeichnet, dass** zusätzlich zu dem mindestens einen Siliconöl c) ii), ausgewählt aus Dimethicone und Cyclomethicone, und zusätzlich zu dem mindestens einen Kohlenwasserstofföl c) iii) mindestens ein weiteres kosmetisches Öl, bevorzugt in einer Gesamtmenge von 0,1 - 5 Gew.-%, besonders bevorzugt 0,3 - 3 Gew.-%, außerordentlich bevorzugt 0,5 - 1 Gew.-%, bezogen auf das Gewicht der Antitranspirant-Zusammensetzung, enthalten ist.

9. Zusammensetzung nach einem der Ansprüche 1 bis 8, **dadurch gekennzeichnet, dass** Wasser in einer Gesamtmenge von 40 - 90 Gew.-%, bevorzugt 50 - 85 Gew.-%, besonders bevorzugt 60 - 80 Gew.-%, außerordentlich bevorzugt 65 - 75 Gew.-%, jeweils bezogen auf das Gewicht der Zusammensetzung, enthalten ist.

10. Zusammensetzung nach einem der Ansprüche 1 bis 9, **dadurch gekennzeichnet, dass** mindestens eine schweißhemmende Aluminium-Verbindung in einer Gesamtmenge von 5 - 35 Gew.-%, bevorzugt 8 - 30 Gew.-%, besonders bevorzugt 10 - 25 Gew.-% und außerordentlich bevorzugt 16 - 20 Gew.-%, jeweils bezogen auf das Gesamtgewicht der kristallwasser- und ligandenfreien Aktivsubstanz (USP) in der Gesamtzusammensetzung, enthalten ist.

11. Zusammensetzung nach einem der Ansprüche 1 bis 9, **dadurch gekennzeichnet, dass** mindestens eine schweißhemmende Aluminium-Zirconium-Verbindung in einer Gesamtmenge von 5 - 35 Gew.-%, bevorzugt 8 - 30 Gew.-%, besonders bevorzugt 10 - 25 Gew.-% und außerordentlich bevorzugt 16 - 20 Gew.-%, jeweils bezogen auf das Gesamtgewicht der kristallwasser- und ligandenfreien Aktivsubstanz (USP) in der Gesamtzusammensetzung, enthalten ist.

12. Zusammensetzung nach einem der Ansprüche 1 bis 11, **dadurch gekennzeichnet, dass** sie in Form einer Öl-in-Wasser-Emulsion vorliegt und Steareth-2, Steareth-21 und PPG-15-Stearyl-ether enthalten sind.

13. Nicht-therapeutisches Verfahren zur schweißhemmenden Behandlung der Haut, **dadurch gekennzeichnet, dass** eine Antitranspirant-Zusammensetzung nach einem der Ansprüche 1 bis 12 auf die Haut appliziert wird.

14. Verwendung einer Mischung, bestehend aus:
i. mindestens einem Siliconelastomer,
ii. mindestens einem unter Normalbedingungen flüssiges Siliconöl, ausgewählt aus Dimethicone und Cyclomethicone, sowie
iii. mindestens einem nur aus C und H bestehenden Kohlenwasserstofföl,
zur Reduzierung der Adhäsivität einer Antitranspirant-Zusammensetzung zur Roll-on-Applikation auf die Haut, enthaltend Wasser und mindestens eine schweißhemmende Aluminium- oder Aluminium-Zirconium-Verbindung, auf der Haut, wobei die Antitranspirant-Zusammensetzung kein Copolymer, bestehend aus mindestens zwei Monomeren, ausgewählt aus der Gruppe, die gebildet wird aus Ethylen, Propylen, Buten und Styren, enthält.

15. Verwendung nach Anspruch 14, **dadurch gekennzeichnet, dass** die Antitranspirant-Zusammensetzung eine Antitranspirant-Zusammensetzung nach einem der Ansprüche 2 bis 12 ist.

## Claims

1. An antiperspirant composition for roll-on application, containing:
a. water,
b. at least one perspiration-inhibiting aluminum or aluminum-zirconium compound,
c. an oil phase, containing:
i. at least one silicone elastomer,
ii. at least one silicone oil that is liquid under normal conditions, selected from dimethicone and cyclomethicone, and
iii. at least one hydrocarbon oil consisting of only C and H,
wherein the composition does not contain any copolymer consisting of at least two monomers selected from the group consisting of ethylene, propylene, butene and styrene.

2. The composition according to claim 1, **characterized in that** the at least one silicone elastomer i) is selected from vinyl dimethicone/methicone silsesquioxane crosspolymer, dimethicone/vinyl dimethicone crosspolymer, dimethicone/phenyl vinyl dimethicone crosspolymer, stearyl-vinyl/hydromethylsiloxane copolymer, polysilicone-11, stearoxymethicone/dimethicone copolymer, and mixtures thereof, preferably selected from vinyl dimethicone/methicone silsesquioxane crosspolymer.

3. The composition according to one of claims 1 or 2, **characterized in that** the at least one hydrocarbon oil iii) is selected from mineral oil, paraffin oil, polyisobutene, hydrogenated polyisobutene, polydecene, hydrogenated polydecene, C8-C20 isoparaffins such as isononane, isodecane, isoundecane, isododecane, isotridecane, isotetradecane, isopentadecane, isohexadecane and isoeicosane, and C10-C20 n-alkanes, preferably C13-C15 n-alkanes, and mixtures thereof.

4. The composition according to one of claims 1 to 3, **characterized in that** the at least one silicone elastomer i) is contained in a total amount of from 0.01 to 2 wt.%, preferably 0.02 to 1 wt.%, particularly preferably 0.05 to 0.2 wt.%, in each case based on the weight of the antiperspirant composition.

5. The composition according to one of claims 1 to 4, **characterized in that** the at least one silicone oil ii), selected from dimethicone and cyclomethicone, is contained in a total amount of from 0.05 to 5 wt.%, preferably 0.1 to 3 wt.%, particularly preferably 0.8 to 2 wt.%, in each case based on the weight of the antiperspirant composition.

6. The composition according to one of claims 1 to 5, **characterized in that** the at least one hydrocarbon oil iii) consisting of only C and H is contained in a total amount of from 0.01 to 10 wt.%, preferably 0.1 to 5 wt.%, particularly preferably 0.5 to 2 wt.%, extremely preferably 1.0 to 1.5 wt.%, in each case based on the weight of the antiperspirant composition.

7. The composition according to one of claims 1 to 6, **characterized in that** at least one oil-in-water emulsifier is contained having an HLB value of greater than 7 to 20, preferably at least one non-ionic oil-in-water emulsifier having an HLB value of greater than 7 to 20, and optionally at least one water-in-oil emulsifier is contained having an HLB value of greater than 1.0 and less than/equal to 7.0, preferably in the range of from 3 to 6.

8. The composition according to one of claims 1 to 7, **characterized in that**, in addition to the at least one silicone oil c) ii), selected from dimethicone and cyclomethicone, and in addition to the at least one hydrocarbon oil c) iii), at least one additional cosmetic oil is contained, preferably in a total amount of from 0.1 to 5 wt.%, particularly preferably 0.3 to 3 wt.%, extremely preferably 0.5 to 1 wt.%, based on the weight of the antiperspirant composition.

9. The method according to one of claims 1 to 8, **characterized in that** water is contained in a total amount of from 40 to 90 wt.%, preferably 50 to 85 wt.%, particularly preferably 60 to 80 wt.% extremely preferably 65 to 75 wt.%, in each case based on the weight of the composition.

10. The composition according to one of claims 1 to 9, **characterized in that** at least one perspiration-inhibiting aluminum compound is contained in a total amount of from 5 to 35 wt.%, preferably 8 to 30 wt.%, particularly preferably 10 to 25 wt.% and extremely preferably 16 to 20 wt.%, in each case based on the total weight of the active substance (USP), which is free of water of crystallization and ligands, in the total composition.

11. The composition according to one of claims 1 to 9, **characterized in that** at least one perspiration-inhibiting aluminum-zirconium compound is contained in a total amount of from 5 to 35 wt.%, preferably 8 to 30 wt.%, particularly preferably 10 to 25 wt.% and extremely preferably 16 to 20 wt.%, in each case based on the total weight of the active substance (USP), which is free of water of crystallization and ligands, in the total composition.

12. The composition according to one of claims 1 to 11, **characterized in that** it is in the form of an oil-in-water emulsion, and steareth-2, steareth-21 and PPG-15 stearyl ether are contained.

13. A non-therapeutic method for treating the skin in a manner inhibiting perspiration, **characterized in that** an antiperspirant composition according to one of claims 1 to 12 is applied to the skin.

14. The use of a mixture consisting of:
i. at least one silicone elastomer,
ii. at least one silicone oil that is liquid under normal conditions, selected from dimethicone and cyclomethicone, and
iii. at least one hydrocarbon oil consisting of only C and H,
for reducing the adhesiveness on the skin of an antiperspirant composition for roll-on application to the skin, containing water and at least one perspiration-inhibiting aluminum or aluminum-zirconium compound, wherein the antiperspirant composition does not contain any copolymer consisting of at least two monomers selected from the group consisting of ethylene, propylene, butene and styrene.

15. The use according to claim 14, **characterized in that** the antiperspirant composition is an antiperspirant composition according to one of claims 2 to 12.

## Revendications

1. Composition anti-transpirante pour application à bille, comprenant :
a. de l'eau
b. au moins un composé d'aluminium ou d'aluminium-zirconium anti-transpirant,
c. une phase huileuse contenant :
i. au moins un élastomère de silicone,
ii. au moins une huile de silicone liquide dans des conditions normales choisie parmi la diméthicone et la cyclométhicone, et
iii. au moins une huile hydrocarbonée constituée uniquement de C et de H,
la composition ne contenant pas de copolymère constitué d'au moins deux monomères choisis dans le groupe constitué par l'éthylène, le propylène, le butène et le styrène.

2. Composition selon la revendication 1, **caractérisée en ce que** l'au moins un élastomère de silicone i) est choisi parmi le polymère réticulé vinyldiméthicone/méthicone silsesquioxane, , le polymère réticulé diméthicone/vinyldiméthicone, le polymère réticulé diméthicone/phénylvinyldiméthicone, le copolymère stéaryl-vinyl/hydrométhylsiloxane, le polysilicone-11, le copolymère stéaroxyméthicone/diméthicone et leurs mélanges, de préférence il est choisi parmi le polymère réticulé vinyldiméthicone/méthicone silsesquioxane.

3. Composition selon l'une des revendications 1 ou 2, **caractérisée en ce que** l'au moins une huile hydrocarbonée iii) est choisie parmi une huile minérale, une huile de paraffine, le polyisobutène, le polyisobutène hydrogéné, le polydécène, le polydécène hydrogéné, les isoparaffines en C₈ à C₂₀ telles que l'isononane, l'isodécane, l'isoundécane, l'isododécane, l'isotridécane, l'isotétradécane, l'isopentadécane, l'isohexadécane et l'isoeicosane, ainsi que les n-alcanes en C₁₀ à C₂₀, de préférence les n-alcanes en C₁₃ à C₁₅, ainsi que leurs mélanges.

4. Composition selon l'une des revendications 1 à 3, **caractérisée en ce que** l'au moins un élastomère de silicone i) est contenu dans une quantité totale de 0,01 à 2 % en poids, de préférence de 0,02 à 1 % en poids, de manière particulièrement préférée de 0,05 à 0,2 % en poids, à chaque fois sur la base du poids de la composition anti-transpirante.

5. Composition selon l'une des revendications 1 à 4, **caractérisée en ce que** l'au moins une huile de silicone ii), choisie parmi la diméthicone et la cyclométhicone, est contenue dans une quantité totale de 0,05 à 5 % en poids, de préférence de 0,1 à 3 % en poids, de manière particulièrement préférée de 0,8 à 2 % en poids, par rapport au poids de la composition anti-transpirante.

6. Composition selon l'une des revendications 1 à 5, **caractérisée en ce que** l'au moins une huile hydrocarbonée (iii), constituée uniquement de C et de H, est contenue dans une quantité totale de 0,01 à 10 % en poids, de préférence de 0,1 à 5 % en poids, de manière particulièrement préférée de 0,5 à 2 % en poids, de manière extraordinairement préférée de 1,0 à 1,5 % en poids, à chaque sur la base du poids de la composition anti-transpirante.

7. Composition selon l'une des revendications 1 à 6, **caractérisée en ce qu'**au moins un émulsifiant huile-dans-eau ayant une valeur HLB supérieure à la gamme de 7 à 20, de préférence au moins un émulsifiant huile-dans-eau non ionique ayant une valeur HLB supérieure à la gamme de 7 à 20, et éventuellement au moins un émulsifiant eau-dans-huile ayant une valeur HLB supérieure à 1,0 et inférieure ou égale à 7,0, de préférence dans la gamme de 3 à 6.

8. Composition selon l'une des revendications 1 à 7, **caractérisée en ce que**, en plus de l'au moins une huile de silicone c) ii) choisie parmi la diméthicone et la cyclométhicone, et en plus de l'au moins une huile hydrocarbonée c) iii), au moins une autre huile cosmétique est contenue de préférence dans une quantité totale de 0,1 à 5 % en poids, de manière particulièrement préférée de 0,3 à 3 % en poids, de manière extraordinairement préférée de 0,5 à 1 % en poids, sur la base du poids de la composition anti-transpirante.

9. Composition selon l'une des revendications 1 à 8, **caractérisée en ce que** de l'eau est contenue dans une quantité totale de 40 à 90 % en poids, de préférence de 50 à 85 % en poids, de manière particulièrement préférée de 60 à 80 % en poids, de manière extraordinairement préférée de 65 à 75 % en poids, à chaque sur la base du poids de la composition.

10. Composition selon l'une des revendications 1 à 9, **caractérisée en ce qu'**au moins un composé d'aluminium anti-transpirant est contenu dans une quantité totale de 5 à 35 % en poids, de préférence de 8 à 30 % en poids, de manière particulièrement préférée de 10 à 25 % en poids et de manière extraordinairement préférée de 16 à 20 % en poids, à chaque fois sur la base du poids total de la substance active sans ligand ni eau de cristallisation (USP) dans la composition totale.

11. Composition selon l'une des revendications 1 à 9, **caractérisée en ce qu'**au moins un composé d'aluminium-zirconium anti-transpirant est contenu dans une quantité totale de 5 à 35 % en poids, de préférence de 8 à 30 % en poids, de manière particulièrement préférée de 10 à 25 % en poids et de manière extraordinairement préférée de 16 à 20 % en poids, à chaque fois sur la base du poids total de la substance active sans ligand ni eau de cristallisation (USP) dans la composition totale.

12. Composition selon l'une des revendications 1 à 11, **caractérisée en ce qu'**elle se présente sous la forme d'une émulsion huile-dans-eau et comprend du stéareth-2, du stéareth-21 et du PPG-15-stéaryl-éther.

13. Procédé non thérapeutique pour le traitement anti-transpirant de la peau, **caractérisé en ce qu'**une composition anti-transpirante selon l'une des revendications 1 à 12 est appliquée sur la peau.

14. Utilisation d'un mélange constitué :
i. d'au moins un élastomère de silicone,
ii. d'au moins une huile de silicone liquide dans des conditions normales choisie parmi la diméthicone et la cyclométhicone, et
iii. au moins une huile hydrocarbonée constituée uniquement de C et de H,
pour réduire l'adhésivité sur la peau d'une composition anti-transpirante, destinée à l'application sur la peau avec un dispositif à bille, contenant de l'eau et au moins un composé d'aluminium ou d'aluminium-zirconium anti-transpirant, la composition anti-transpirante ne contenant pas de copolymère constitué d'au moins deux monomères choisis dans le groupe constitué par l'éthylène, le propylène, le butène et le styrène.

15. Utilisation selon la revendication 14, **caractérisée en ce que** la composition anti-transpirante est une composition anti-transpirante selon l'une des revendications 2 à 12.
